# EUROPEAN PATENT APPLICATION

(11) **EP 4 776 198 A1**
(43) Date of publication of application: **15.07.2026**
(21) Application number: 23951575.2
(22) Date of filing: 08.09.2023
(51) Int. Cl.: G06Q 10/06

(54) **OUTPUT PROGRAM, OUTPUT METHOD, AND INFORMATION PROCESSING DEVICE**

(71) Applicant: FUJITSU LIMITED, Kawasaki-shi, Kanagawa 211-8588 (JP)
(72) Inventor: NAKAYAMA, Sayuri, Kawasaki-shi, Kanagawa 211-8588 (JP); INOMATA, Akihiro, Kawasaki-shi, Kanagawa 211-8588 (JP); TADA, Atsuko, Kawasaki-shi, Kanagawa 211-8588 (JP); SASAMOTO, Yuki, Kawasaki-shi, Kanagawa 211-8588 (JP)
(74) Representative: Hoffmann Eitle
(86) International application number: PCT/JP2023/032926
(87) International publication number: WO 2025/052677

(57) **Abstract**

An output program causes a computer to execute processing of receiving information related to a policy, identifying a policy model composed of a plurality of components defining a detail related to the policy in a hierarchical structure by using the received information related to the policy, generating a policy model in which one or more components corresponding to the information related to the policy among the components of the identified policy model are changed, and outputting the generated policy model.

## Description

### Technical Field

The present invention relates to an output program, an output method, and an information processing device.

### Background Art

As one workflow, a policy flow graph is known that schematically expresses a flow of allocation of objects, such as people, being a target of a policy to services for achieving an objective of the policy in various fields, such as medical care, nursing care, and public administration.

For example, to generate the policy flow graph, artificial intelligence (AI) chat services implemented by large language models, so-called LLM, such as Transformer, can be used.

Some of these AI chat services output a flowchart by using text as input. For example, by providing a prompt to set a role, such as an interviewer, with preconditions, detailed conditions, and the like defined, a flow can be output through an interview format.

### Citation List

### Patent Literature

Patent Literature 1: Japanese Laid-open Patent Publication No. 2003-228647

### Summary of invention

### Technical Problem

Unfortunately, the above AI chat services have such an aspect that it is difficult to generate a policy flow graph conforming to the site to which the policy is applied. For example, the above AI chat services may output a policy flow graph that lacks service, condition, or other components indispensable for the site or that contains service, conditional branch, or other components not meeting standards, such as laws, regulations, and guidelines.

Note that the above problem is not limited to a policy model implemented in a flow format, such as the policy flow graph, but is a problem arising similarly in any policy model implemented in social concept, organization chart, guideline, and other formats.

In one aspect, an object is to provide an output program, an output method, and an information processing device that enable generation of a policy model conforming to a site to which a policy is applied.

### Solution to Problem

According to an aspect of an embodiment, an output program causes a computer to execute processing including: receiving information related to a policy; identifying a policy model by using the received information related to the policy, the policy model being composed of a plurality of components defining a detail related to the policy in a hierarchical structure; generating a policy model in which one or more components corresponding to the information related to the policy among the components of the identified policy model are changed; and outputting the generated policy model. Advantageous Effects of Invention

According to one embodiment, the generation of the policy model conforming to the site to which the policy is applied is enabled.

### Brief Description of Drawings

FIG. 1 is a block diagram illustrating an example functional configuration of a server device.
FIG. 2 is a diagram exemplifying of a policy flow graph.
FIG. 3 is a diagram illustrating a specific example of the policy flow graph.
FIG. 4 is a diagram illustrating one example of question collection data.
FIG. 5 is a diagram illustrating one example of flow collection data.
FIG. 6 is a schematic view describing one example of association information.
FIG. 7 is a diagram (1) illustrating one example of a question window.
FIG. 8 is a diagram (2) illustrating one example of the question window.
FIG. 9 is a diagram (3) illustrating one example of the question window.
FIG. 10 is a diagram (4) illustrating one example of the question window.
FIG. 11 is a diagram (5) illustrating one example of the question window.
FIG. 12 is a diagram illustrating one example of an answer confirmation window.
FIG. 13 is a diagram illustrating one example of a policy flow generation result.
FIG. 14 is a flowchart illustrating a procedure of model output processing.
FIG. 15 is a block diagram illustrating an example functional configuration of a server device according to an application example.
FIG. 16 is a diagram (1) illustrating one example of a chatbot screen.
FIG. 17 is a diagram (2) illustrating one example of the chatbot screen.
FIG. 18 is a diagram illustrating an example hardware configuration.

### Description of Embodiments

Embodiments of an output program, an output method, and an information processing device according to the present application will be described below with reference to the attached drawings. Each embodiment only describes an example, and this example does not limit the configuration, structure, function, numerical values used to identify these, usage situation, and the like. Each embodiment can be adaptively combined within such a range that processing contents are not contradictory.

### <First Embodiment>

### <System Configuration>

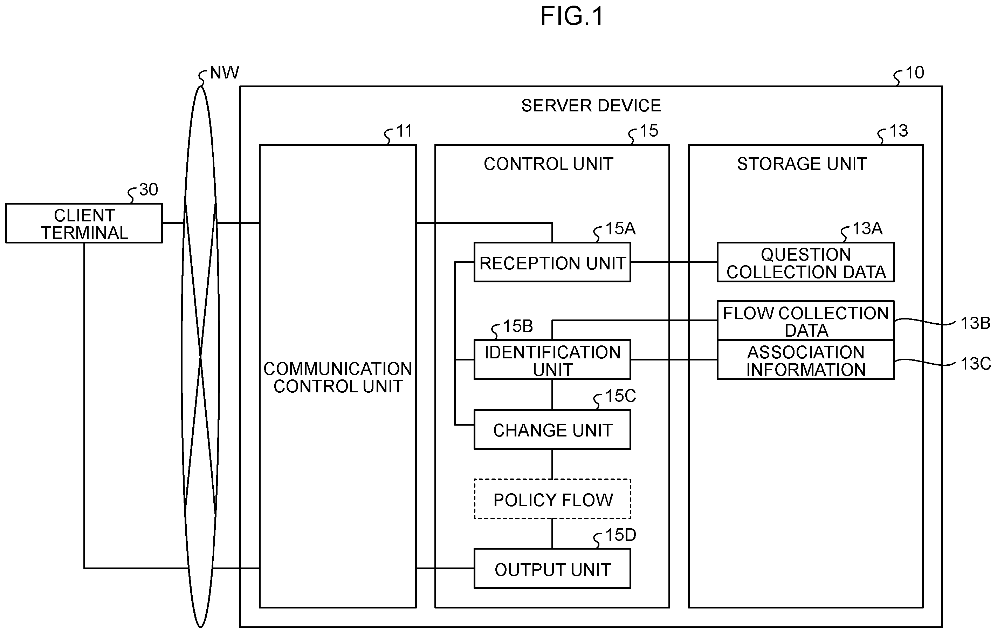

FIG. 1 is a block diagram illustrating an example functional configuration of a server device 10. The server device 10 illustrated in FIG. 1 provides a model output function that outputs a policy model in which a policy design concept, that is, a scenario is digitized, in terms of supporting planning of a policy.

The server device 10 can provide the above model output function as a cloud service by executing Platform-as-a-Service (PaaS)-type middleware or a Software-as-a-Service (SaaS)-type application.

As illustrated in FIG. 1, the server device 10 can be communicatively connected to a client terminal 30 via a network NW. For example, the network NW may be a communication network of any type, such as the Internet or a local area network (LAN), whether it is wired or wireless. Note that FIG. 1 exemplifies connection of one client terminal 30 per server device 10; however, connection of any number of the client terminals 30 is not restrained.

The client terminal 30 is a terminal device that receives the provision of the above model output function. For example, the client terminal 30 can be used by a customer, such as a policy planner, as an example interested person in a subject implementing the policy, for example, a municipality or the like. The client terminal 30 may be implemented by any computer, ranging from a personal computer to a smartphone, a tablet terminal, a wearable terminal, or the like, as an example.

Note that, in the example here, the above model output function is provided as a cloud service; however, no such limitation is intended. For example, the above model output function may be provided on the premises. Furthermore, in the example, the above model output function is provided as a client server system; however, no such limitation is intended. For example, an application running on the client terminal 30 causes the client terminal 30 to execute processing corresponding to the above model output function, whereby the above model output function may be provided on a stand-alone basis.

### <Policy Model>

Herein, "policy model" includes a diagram composed of a plurality of components defining details related to the policy in a hierarchical structure. For example, the policy model can be implemented as a policy flow graph as well as a social concept, an organization chart, a medical guideline, and the like.

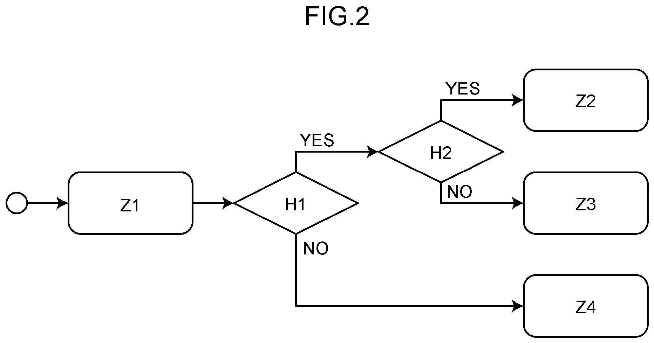

As one policy model, a policy flow graph is exemplified in the following description. FIG. 2 is a diagram exemplifying the policy flow graph. Z1, Z2, Z3, and Z4 illustrated in FIG. 2 indicate, for example, services performed by an administrator to a user. Furthermore, these may be referred to as "service performing components". Specific examples of the services include, for example, having a medical checkup, "intervention" to which an object, such as a resident, being a target of the policy is allocated, like consult by a medical specialist, "no intervention" like follow-up, and the like, taking the medical field as an example, but are not limited to policies in the medical field.

H1 and H2 indicate, for example, conditional branches containing conditions. Furthermore, these may be referred to as "conditional branch components". Specific examples of the conditions include, for example, an estimated glomerular filtration rate (eGFR) below a threshold, a hemoglobin A1c rate (HbA1c) below a threshold, a urinary protein rate equal to or greater than a threshold, and the like, taking the medical field as an example, but are not limited to conditions in the medical field.

Z1, Z2, Z3, and Z4, and H1 and H2 each may be referred to as a "component". These "components" can correspond to one example of "nodes" in terms of graph data. Furthermore, connection between the nodes can correspond to one example of "edges" including a "directed edge" and the like.

Note that, in this embodiment, policy planning in the medical field is described as an example; however, use cases of the above model output function are not limited to this. For example, the above model output function may be applied to various types of policy planning in the transportation field, such as road pricing, and the energy field, such as decarbonization and power supply, as well as for work, a test, a questionnaire, and the like. In that case, the same effect can also be acquired as in the case of application to policy planning in the medical field.

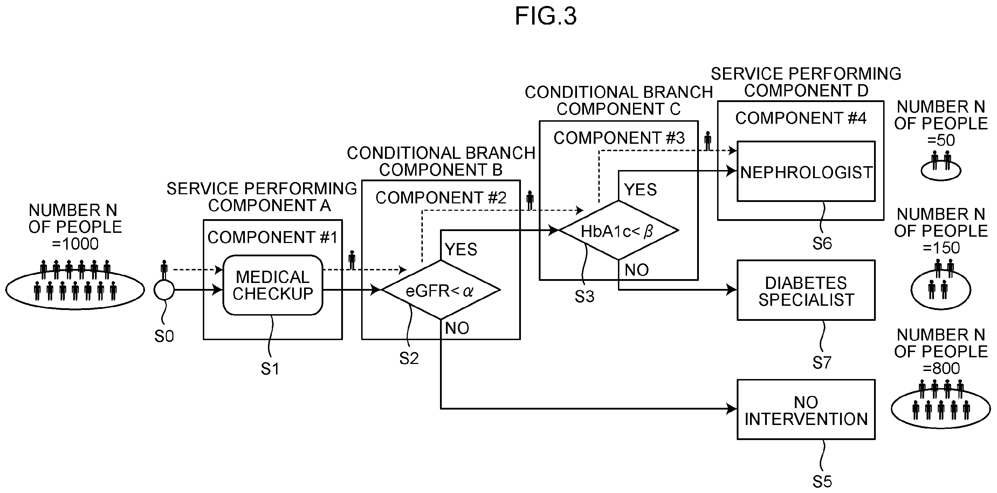

FIG. 3 is a diagram illustrating a specific example of the policy flow graph. As illustrated in FIG. 3, the policy is turned into a model as a workflow composed of a combination of the conditional branch, service performing, and other components. Then, the number of people receiving each service is output from a model trained by accumulating information on the flow of people and a parameter from actual values in use for each conditional branch component.

In the example illustrated in FIG. 3, at sign S0, the number N of people = 1000 is input. At sign S1, a component #1 as a service performing component A is set to "Medical checkup". At sign S2, a component #2 as a conditional branch component B is set to "eGFR < α". If "eGFR < α" is not satisfied (see NO route at the sign S2), it is determined that "No intervention" by a medical specialist is needed for the relevant citizen as illustrated at sign S5.

On the other hand, if "eGFR < α" is satisfied (see YES route at sign S2), a component #3 as a conditional branch component C is set to "HbA1c < β" as illustrated at sign S3. If "HbA1c < β" is satisfied (see YES route at sign S3), a component #4 as a conditional branch component D is set to "Nephrologist" as illustrated at sign S6, and it is determined that intervention by a "Nephrologist" is needed for the relevant citizen. On the other hand, if "HbA1c < β" is not satisfied (see NO route at sign S3), it is determined that intervention by a "Diabetes specialist" is needed for the relevant citizen as illustrated at sign S7.

In the example illustrated in FIG. 3, as illustrated with arrows, the number of people flowing is predicted in the order of the component #1, the component #2, the component #3, and the component #4. For example, the following is a result of allocating the number N of people = 1000 among Z2 to Z4 being intervention in the policy flow graph illustrated in FIG. 3. To the intervention Z2, 50 people are allocated. To the intervention Z3, 150 people are allocated. Furthermore, to the intervention Z4, 800 people are allocated.

Hereinafter, the policy flow graph may be abbreviated as a "policy flow". The policy flow may be shared in any framework. Only as an example, the policy flow can be shared among organizations around the world, for example, municipalities and other public bodies, through a data infrastructure platform where the policy flow can be shared, cross-referenced, and updated.

Such a data infrastructure platform may be provided by a business entity providing the above model output function or may be provided by another business entity. For example, the policy planner can reference to a policy flow around the world collected in the above data infrastructure via the client terminal 30. At this time, by incorporating the entire or part of the policy flow collected in the data infrastructure, an existing policy flow can be updated to support planning of a draft policy flow.

Moreover, the above data infrastructure can provide not only sharing of the policy flow but also the following back-end functions. For example, a simulation of simulating the flow of an object being a target of a service on existing and draft policy flows can be performed. Furthermore, evaluation of each index, such as effect and cost, in existing and draft policy flows, and comparison between existing and draft policy flows or comparison between a plurality of draft policy flows can be performed.

### <Configuration of Server Device 10>

Next, the functional configuration of the server device 10 providing the above model output function will be described. FIG. 1 schematically illustrates blocks related to the model output function of the server device 10. As illustrated in FIG. 1, the server device 10 includes a communication control unit 11, a storage unit 13, and a control unit 15. Note that FIG. 1 only illustrates selected functional units related to the above model output function, and the server device 10 may include a functional unit other than those illustrated in the drawing.

The communication control unit 11 is a functional unit controlling communication with another device, such as the client terminal 30. Only as an example, the communication control unit 11 can be implemented by a network interface card, such as a LAN card. In one aspect, the communication control unit 11 receives a policy flow generation request from the client terminal 30 or outputs the policy flow to the client terminal 30.

The storage unit 13 is a functional unit storing various pieces of data therein. Only as an example, the storage unit 13 may be implemented by an internal, external, or auxiliary storage of the server device 10. For example, the storage unit 13 stores question collection data 13A, flow collection data 13B, and association information 13C. Note that the question collection data 13A, the flow collection data 13B, and the association information 13C are only examples, and storing data other than these in the storage unit 13 is not restrained.

The question collection data 13A may be a set of question templates in which questions related to the policy are turned into patterns. These question templates may contain a question for receiving specification of a type of policy to be planned in a set of flow templates in which a basic form of the policy flow is turned into a pattern for each type of policy. Moreover, the question templates may contain a question for customizing part of the flow template.

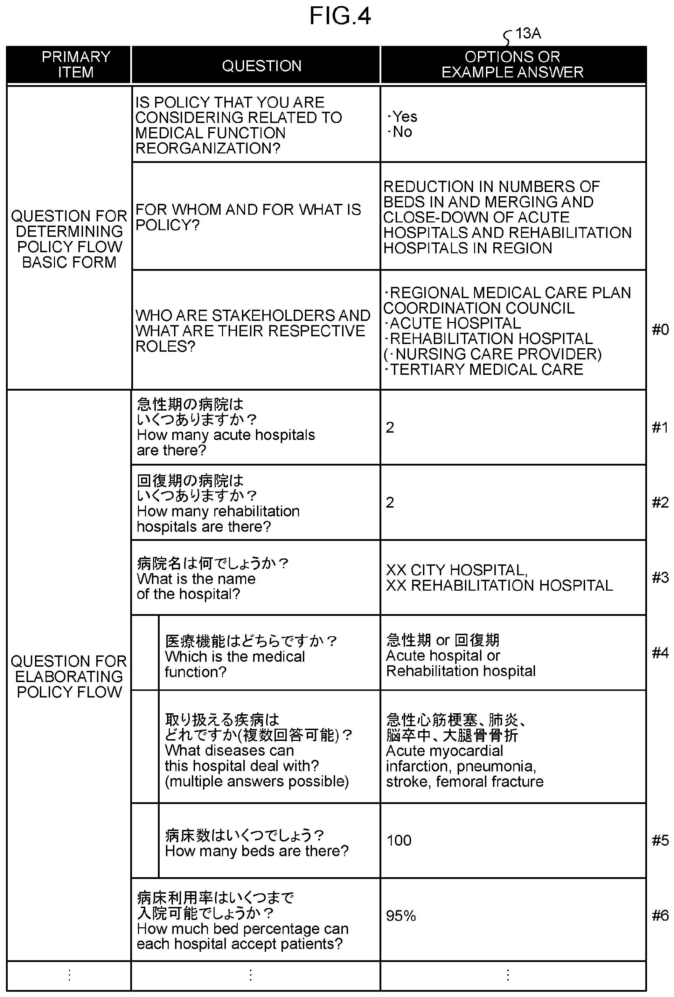

FIG. 4 is a diagram illustrating one example of the question collection data 13A. As illustrated in FIG. 4, the question collection data 13A may be data in which items, such as "Primary item", "Question", and "Options or example answer", are associated with each other.

Herein, "Primary item" indicates items classifying categories of the questions. Furthermore, "Question" indicates questions or text corresponding to definition of questions. Furthermore, "Options or example answer" indicates answer options or example answers to questions. Note that "Options or example answer" is illustrated in the drawing for convenience of description but does not always need to be used to present a question or acquire an answer.

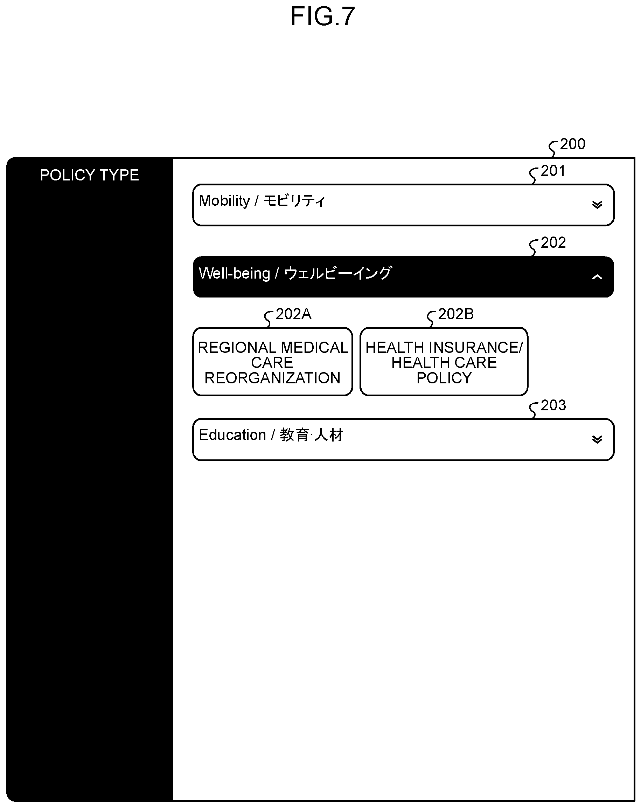

For example, FIG. 4 illustrates, only as an example of the policy type, selected question template collection related to medical function reorganization. Although details are described later using FIGS. 7 to 12, questions are presented on the client terminal 30 on the basis of the question templates illustrated in FIG. 4, whereby specification of the policy type identifying a type of policy to be planned, details of customization of a flow template, and the like can be received as answers.

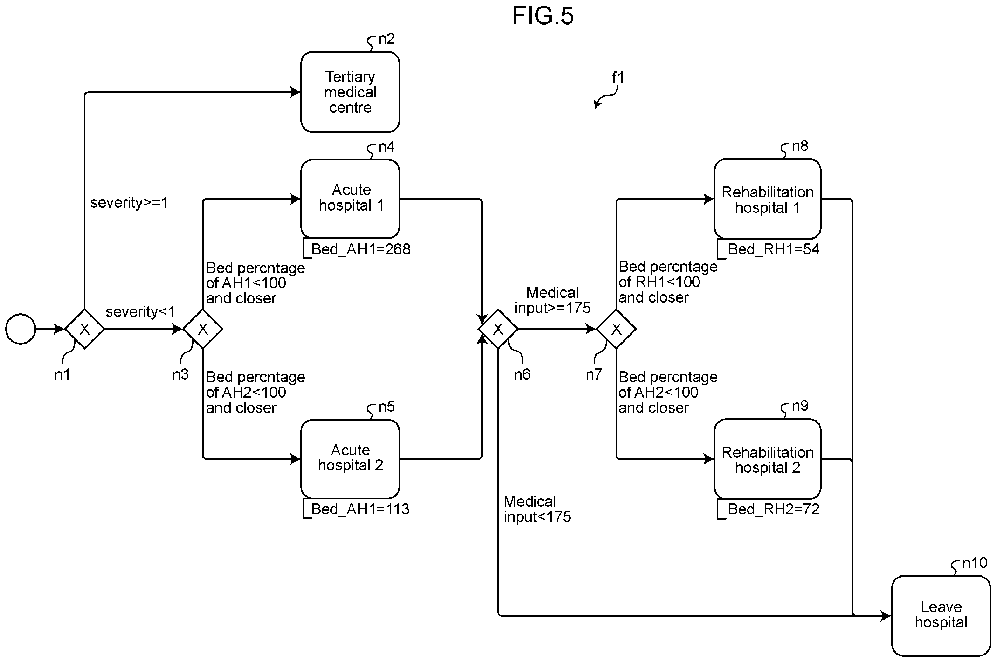

The flow collection data 13B may be a set of flow templates in which the basic forms of policy flows are turned into patterns. FIG. 5 is a diagram illustrating one example of the flow collection data 13B. FIG. 5 illustrates, only as an example of the flow templates, a selected flow template f1 in which the basic form of a policy flow for a policy type "medical function reorganization" is turned into a pattern. As illustrated in FIG. 5, in the flow template f1, a procedure for allocating an object, such as a resident, to a service component corresponding to each medical function in the order of the highly acute phase, the acute phase, and the rehabilitation phase is written in Business Process Model and Notation (BPMN) format.

The association information 13C is information defining association of the question templates and the flow templates. Only as an example, in the association information 13C, association between the question templates and target elements, for example, service, conditional branch, and other components or parameters of the components customized in accordance with answers corresponding to the question templates in the flow template may be written. Such association may be written in any format, such as Java (registered trademark) Script Object Notation (JSON) or a markup language, only as an example.

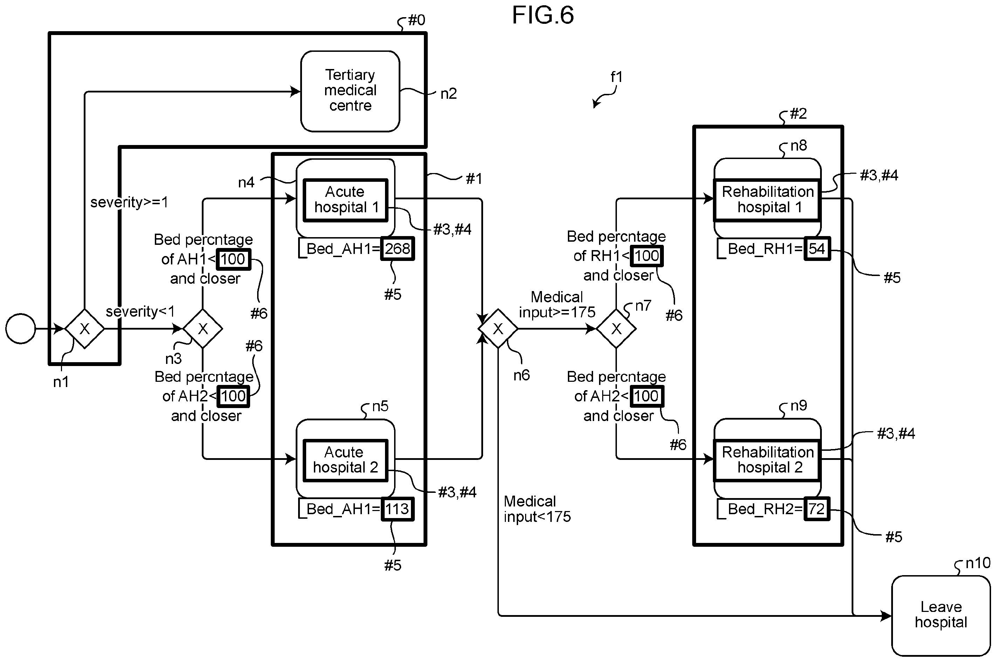

FIG. 6 is a schematic view describing one example of the association information 13C. In FIG. 6, related elements #0 to #6 related to the question templates contained in the question collection data 13A illustrated in FIG. 4 in the flow template f1 illustrated in FIG. 5 are illustrated in bold frames.

The association information 13C may include the related elements #0 to #6 illustrated in FIG. 6. Furthermore, the related elements #0 to #6 may be correlated to information on a modification pattern for customizing part of the flow template in accordance with answers corresponding to the question templates. Such a modification pattern may include the following four modification patterns P1 to P4, only as an example.

As one aspect, the related elements #1 to #4 are correlated to the modification pattern P1 for customizing service components arranged in parallel on the flow template or parameters of the components.

For example, in the example of the flow template f1, examples of the service components arranged in parallel on the flow template f1 include acute medical institutions and rehabilitation medical institutions. In this flow template f1, as example acute medical institutions, two components n4 and n5 are illustrated, and, as example rehabilitation medical institutions, two components n4 and n5 are illustrated; however, the number of these medical institutions can be changed in accordance with the modification pattern P1.

To be specific, in the example of the related element #1, the number of the acute medical institutions arranged in parallel on the flow template f1 is changed in accordance with an answer corresponding to the question template of the related element #1. Furthermore, in the example of the related element #2, the number of the rehabilitation medical institutions arranged in parallel on the flow template f1 is changed in accordance with an answer corresponding to the question template of the related element #2.

Furthermore, in the example of the flow template f1, examples of the parameters of the service components arranged in parallel on the flow template f1 include the names and medical functions of the medical institutions. These parameters can also be changed in accordance with the modification pattern P1.

To be specific, in the example of the related element #3, the names of the acute medical institutions n4 and n5 or the names of the rehabilitation medical institutions n8 and n9 are set in accordance with answers corresponding to the question template of the related element #3. Furthermore, in the example of the related element #4, the medical functions, that is, the acute phase or the rehabilitation phase, of the medical institutions n4, n5, n8, and n9 are selected in accordance with answers corresponding to the question template of the related element #4.

As another aspect, the related element #5 is correlated to the modification pattern P2 for changing meta-information on a service component included in the flow template. For example, in the example of the related element #5, as example detailed information on the medical institutions n4, n5, n8, and n9, the numbers of beds are set in accordance with answers corresponding to the question template of the related element #5. Note that the number of beds is only an example item included as the detailed information of the medical institution, and another item, such as the number of doctors, may be used.

As a further aspect, the related element #6 is correlated to the modification pattern P3 for changing a parameter of a conditional branch component included in the flow template. For example, in the example of the related element #6, the thresholds of the bed occupancy rates used in determination of a conditional branch component n3 and a conditional branch component n7 are set in accordance with answers corresponding to the question template of the related element #6.

As another aspect, the related element #0 is correlated to the modification pattern P4 for deleting a conditional branch component included in the flow template, or a conditional branch component included in the flow template and a service component connected to that component. For example, in the example of the related element #0, a conditional branch component n1 and a service connected to the conditional branch component n1, that is, a tertiary medical care component n2 are set in accordance with an answer corresponding to the question template of the related element #0.

In this way, in the example illustrated in FIG. 6, the association information 13C may contain the related element #0 (modification pattern P4), the related elements #1 to #4 (modification pattern 1), the related element #5 (modification pattern P2), the related element #6 (modification pattern P3), and the like.

The control unit 15 is a functional unit performing overall control of the server device 10. Only as an example, the control unit 15 can be implemented by a hardware processor. Alternatively, the control unit 15 may be implemented by hardwired logic. As illustrated in FIG. 1, the control unit 15 includes a reception unit 15A, an identification unit 15B, a change unit 15C, and an output unit 15D.

The reception unit 15A is a processing unit receiving various pieces of information from the client terminal 30. Only as an example, the reception unit 15A can receive the policy flow generation request from the client terminal 30. If receiving this policy flow generation request, the reception unit 15A can receive various pieces of information related to a policy to be planned.

As one aspect, the reception unit 15A presents a question eliciting an answer on a flow template, a so-called flow pattern, on the client terminal 30.

FIG. 7 is a diagram (1) illustrating one example of a question window. FIG. 7 illustrates a question window 200 generated in accordance with a question template belonging to the primary item "Question for determining policy flow basic form" in the question collection data 13A illustrated in FIG. 4.

As illustrated in FIG. 7, the question window 200 includes pull-down menus 201, 202, and 206. These pull-down menus 201, 202, and 206 correspond to primary categories "Mobility", "Well-being", and "Education/Human resource" of the policy type.

At this time, if operation to any pull-down menu among the pull-down menus 201, 202, and 206 is performed, an item button corresponding to the pull-down menu to which operation is performed is displayed.

Only as an example, FIG. 7 illustrates an example in which the pull-down menu 202 is selected. In this case, item buttons 202B and 202C corresponding to the pull-down menu are displayed. These item buttons 202B and 202C correspond to secondary categories "Regional medical care reorganization" and "Health insurance/Health care policy" of the policy type. By receiving operation to any item button between these item buttons 202B and 202C, specification of the policy type can be received.

As another aspect, the reception unit 15A presents a question eliciting an answer on details of customization of the flow template of which the specification is received from the client terminal 30, on the client terminal 30.

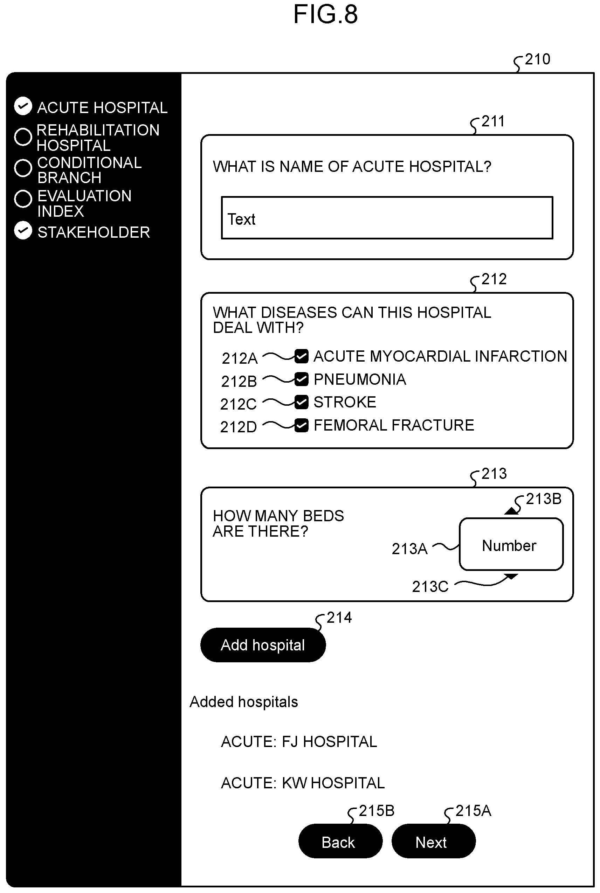

FIG. 8 is a diagram (2) illustrating one example of the question window. FIG. 8 illustrates a question window 210 generated in accordance with a question template belonging to the primary item "Question for elaborating policy flow" in the question collection data 13A illustrated in FIG. 4.

Only as an example, if operation to the item button 202B for the policy type "Regional medical care reorganization" on the question window 200 is performed, a transition from the question window 200 to the question window 210 may be made.

As illustrated in FIG. 8, on the question window 210, question display fields 211 to 213 containing questions related to customization, in other words, processing, editing, and the like, related to an acute hospital are displayed.

Among these, the question display field 211 is generated on the basis of the question template "What is the name of the hospital?" in the question collection data 13A illustrated in FIG. 4. Furthermore, the question display field 211 contains a textbox 211A. Via text input to this textbox 211A, an answer on the name of the acute hospital can be received.

Furthermore, the question display field 212 is generated on the basis of the question template "What diseases can this hospital deal with? (multiple answers possible)" in the question collection data 13A illustrated in FIG. 4. Furthermore, the question display field 212 contains four checkboxes 212A to 212D. Via selection operation to these checkboxes 212A to 212D, an answer on the type of one or a plurality of diseases that the acute hospital can deal with can be received.

Furthermore, the question display field 213 is generated on the basis of the question template "How many beds are there?" in the question collection data 13A illustrated in FIG. 4. Furthermore, the question display field 213 contains a textbox 213A receiving input of a numerical value, a spin button 213B receiving an increase in the numerical value, and a spin button 213C receiving a decrease in the numerical value. Via a spin box including these textbox 213A, spin button 213B, and spin button 213C, an answer on the number of beds in the acute hospital can be received.

If operation to a GUI button 214, "Add hospital", is performed with answers input to these question display fields 211 to 213, a request to add an acute hospital corresponding to the answers to the question display fields 211 to 213 as a customization target of the flow template is received.

Only as an example, FIG. 8 illustrates an example in which "FJ Hospital" and "KW Hospital" are added as example customization targets of the flow template. If operation to a GUI button 215A, "Next", is performed with the customization targets of the flow template registered in this way, a transition to presentation of a subsequent window is made. Note that, if operation to a GUI button 215B, "Back", is performed, presentation returns to the previous window.

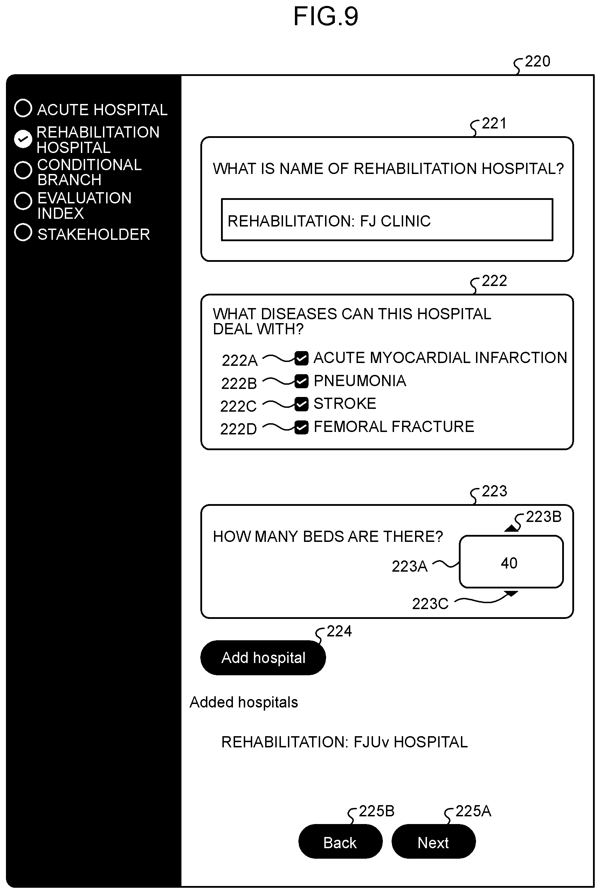

FIG. 9 is a diagram (3) illustrating one example of the question window. FIG. 9 illustrates a question window 220 generated in accordance with a question template belonging to the primary item "Question for elaborating policy flow" in the question collection data 13A illustrated in FIG. 4.

As illustrated in FIG. 9, on the question window 220, question display fields 221 to 223 containing questions related to customization, in other words, processing, editing, and the like, related to a rehabilitation hospital are displayed.

Among these, the question display field 221 is generated on the basis of the question template "What is the name of the hospital?" in the question collection data 13A illustrated in FIG. 4. Furthermore, the question display field 221 contains a textbox 221A. Via text input to this textbox 221A, an answer on the name of the rehabilitation hospital can be received.

Furthermore, the question display field 222 is generated on the basis of the question template "What diseases can this hospital deal with? (multiple answers possible)" in the question collection data 13A illustrated in FIG. 4. Furthermore, the question display field 222 contains four checkboxes 222A to 222D. Via selection operation to these checkboxes 222A to 222D, an answer on the type of one or a plurality of diseases that the rehabilitation hospital can deal with can be received.

Furthermore, the question display field 223 is generated on the basis of the question template "How many beds are there?" in the question collection data 13A illustrated in FIG. 4. Furthermore, the question display field 223 contains a textbox 223A receiving input of a numerical value, a spin button 223B receiving an increase in the numerical value, and a spin button 223C receiving a decrease in the numerical value. Via a spin box including these textbox 223A, spin button 223B, and spin button 223C, an answer on the number of beds in the rehabilitation hospital can be received.

If operation to a GUI button 224, "Add hospital", is performed with answers input to these question display fields 221 to 223, a request to add a rehabilitation hospital corresponding to the answers to the question display fields 221 to 223 as a customization target of the flow template is received.

Only as an example, FIG. 9 illustrates an example in which "FJUv Hospital" and "FJ Clinic" are added as example customization targets of the flow template. If operation to a GUI button 225A, "Next", is performed with the customization targets of the flow template registered in this way, a transition to presentation of a subsequent window is made. Note that, if operation to a GUI button 225B, "Back", is performed, presentation returns to the previous window.

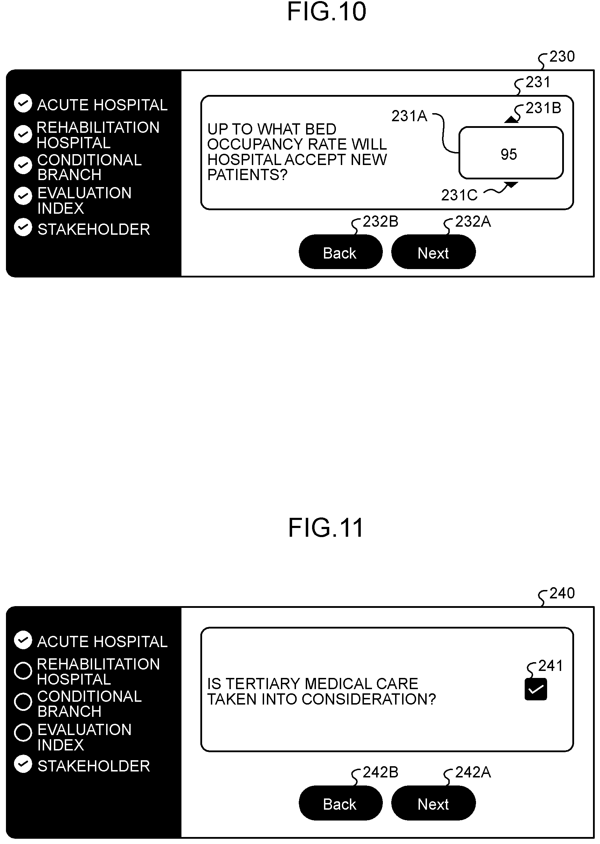

FIG. 10 is a diagram (4) illustrating one example of the question window. FIG. 10 also illustrates a question window 230 generated in accordance with a question template belonging to the primary item "Question for elaborating policy flow" in the question collection data 13A illustrated in FIG. 4.

Only as an example, if operation to the GUI button 225A, "Next", on the question window 220 is performed, a transition from the question window 220 to the question window 230 may be made.

As illustrated in FIG. 10, the question window 230 displays a question display field 231 containing a question eliciting an answer on the bed occupancy rate at which new patients can be accepted in each medical institution of the acute hospital and the rehabilitation hospital. This question display field 231 is generated on the basis of the question template "How much bed percentage can each hospital accept patients?" in the question collection data 13A illustrated in FIG. 4.

Furthermore, the question display field 231 contains a textbox 231A receiving input of a numerical value, a spin button 231B receiving an increase in the numerical value, and a spin button 231C receiving a decrease in the numerical value.

Via a spin box including these textbox 231A, spin button 231B, and spin button 231C, an answer on the bed occupancy rate at which new patients can be accepted can be accepted.

If operation to a GUI button 232A, "Next", is performed with an answer input to the question display field 231 in this way, a transition to presentation of a subsequent window is made. Note that, if operation to a GUI button 232B, "Back", is performed, presentation returns to the previous window.

Note that FIG. 10 exemplifies reception of an answer, common to two medical functions, the acute hospital and the rehabilitation hospital, on the bed occupancy rate at which new patients can be accepted; however, no such limitation is intended. For example, an answer on the bed occupancy rate at which new patients can be accepted in the acute hospital and an answer on the bed occupancy rate at which new patients can be accepted in the rehabilitation hospital may be received separately.

FIG. 11 is a diagram (5) illustrating one example of the question window. FIG. 11 illustrates a question window 240 generated in accordance with a question template belonging to the primary item "Question for determining policy flow basic form" in the question collection data 13A illustrated in FIG. 4.

Only as an example, if operation to the GUI button 232A, "Next", on the question window 230 is performed, a transition from the question window 230 to the question window 240 may be made. This is only an example, the order of displaying the question window 240 is not limited to being subsequent to the question window 230, and a transition may be made subsequent to the question window 200.

As illustrated in FIG. 11, the question window 240 displays a question display field 241 containing a question eliciting an answer on whether a tertiary medical care falls into a category of the policy. This question display field 241 is generated on the basis of the question template "Who are the stakeholders and what are their respective roles?" in the question collection data 13A illustrated in FIG. 4.

Furthermore, the question display field 241 contains a checkbox 241A. Via the presence or absence of a check on this checkbox 241A, an answer on whether the service "Tertiary medical care" component n2 and the conditional branch component n1 allocating an object to the service "Tertiary medical care" component n2 are needed can be received.

Then, if operation to a GUI button 242A, "Next", is performed, a transition to presentation of a subsequent window is made. Note that, if operation to a GUI button 242B, "Back", is performed, presentation returns to the previous window.

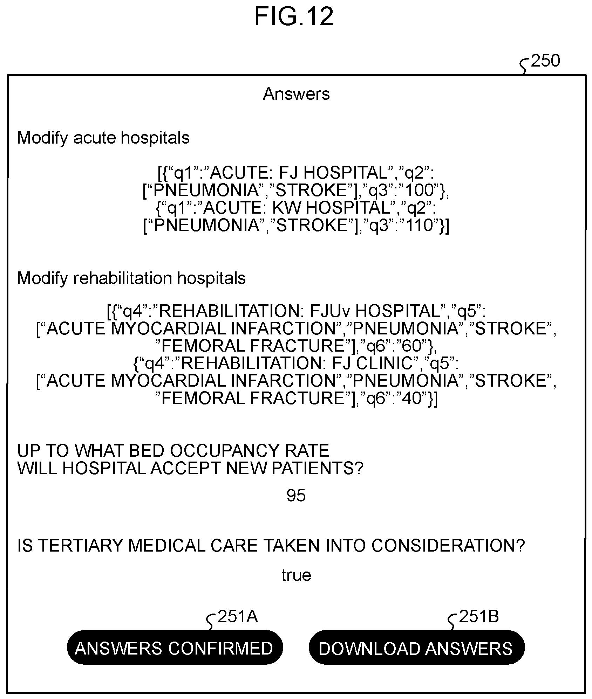

FIG. 12 is a diagram illustrating one example of an answer confirmation window. As illustrated in FIG. 12, on the answer confirmation window 250, display of confirmation of input results of the answers on the question windows 200 to 240 illustrated in FIGS. 7 to 11 is executed.

According to this answer confirmation window 250, confirmation of having received the answer that "FJ Hospital" and "KW Hospital" are added as acute hospital customization targets is displayed. Furthermore, confirmation of having received the answer on the types of diseases that can be dealt with, "Pneumonia" and "Stroke", and having received the answer on the number of beds, "100", as examples of the detailed information on the acute hospital "FJ Hospital" is displayed. In addition, confirmation of having received the answer on the types of diseases that can be dealt with, "Pneumonia" and "Stroke", and having received the answer on the number of beds, "110", as examples of the detailed information on the acute hospital "KW Hospital" is displayed.

Furthermore, according to the answer confirmation window 250, confirmation of having received the answer that "FJUv Hospital" and "FJ Clinic" are added as rehabilitation hospital customization targets is displayed. Furthermore, confirmation of having received the answer on the types of diseases that can be dealt with, "Acute myocardial infarction", "Pneumonia", "Stroke", and "Femoral fracture", and having received the answer on the number of beds, "60", as examples of the detailed information on the rehabilitation hospital "FJUv Hospital" is displayed. In addition, confirmation of having received the answer on the types of diseases that can be dealt with, "Acute myocardial infarction", "Pneumonia", "Stroke", and "Femoral fracture", and having received the answer on the number of beds, "40", as examples of the detailed information on the acute hospital "FJ Clinic" is displayed.

In addition, according to the answer confirmation window 250, confirmation of having received the answer, common to the two medical functions, the acute hospital and the rehabilitation hospital, on the bed occupancy rate at which new patients can be accepted, "95%", as a conditional branch parameter customization target is displayed.

Furthermore, according to the answer confirmation window 250, confirmation of having received the answer on whether a tertiary medical care needs to be considered, "true", as a stakeholder customization target is displayed.

Here, if operation to a GUI button 251A, "Answers confirmed" is performed, customization of the flow template starts. Note that, if operation to a GUI button 251B "Download answers" is performed, a file, for example, a JSON file, in which the input result of the answers displayed for confirmation on the answer confirmation window 250 is written is output to the client terminal 30.

Returning to description of FIG. 1, the identification unit 15B is a processing unit using information related to the policy received by the reception unit 15A to identify a flow template to be planned and a target element to be customized on the flow template. For example, the identification unit 15B receives information related to the policy and identifies a policy model composed of a plurality of components defining details related to the policy in a hierarchical structure by using the received information related to the policy. To be more specific, the identification unit 15B, for example, uses the received information related to the policy to identify a policy model including a diagram composed of the components defining details related to the policy in a hierarchical structure.

As one aspect, the identification unit 15B identifies a flow template corresponding to the policy type of which the specification is received by the reception unit 15A in the flow collection data 13B. Only as an example, the identification unit 15B acquires the policy type of which the specification is received via the question window 200 illustrated in FIG. 7. For example, a case in which the item button 202B for the policy type "Regional medical care reorganization" is selected on the question window 200 illustrated in FIG. 7 is exemplified. In this case, the identification unit 15B identifies the flow template f1 (see FIG. 5) in which the basic form of the policy flow for the policy type "medical function reorganization" is turned into a pattern among the flow templates contained in the flow collection data 13B.

As another aspect, for each answer to the question presented by the reception unit 15A, the identification unit 15B identifies a target element, for example, a service, conditional branch, or other component, or a parameter of the component to be customized in accordance with the answer in the specified flow template. When the target element is identified in this way, the related element in which association of the question template corresponding to the answer is written in the association information 13C stored in the storage unit 13 is referenced.

The change unit 15C is a processing unit changing, for each answer to the question presented by the reception unit 15A, the target element identified by the identification unit 15B in accordance with the modification pattern corresponding to the answer. For example, the change unit 15C generates a policy model in which one or more components corresponding to the information related to the policy among the components of the identified policy model are changed. To be more specific, for example, the change unit 15C identifies one or more components corresponding to the information related to the policy among the components of the identified policy model. Then, the change unit 15C generates a policy model including the diagram in which the identified one or more components are changed on the basis of the received information related to the policy.

Note that the change unit 15C may generate a policy model in which one or more components are changed, by using an answer to a question displayed on a chat screen. For example, the reception unit 15A receives a start instruction of a chat from the terminal 30 and displays a question asking about details of customization of the identified policy model on the chat screen for the chat of which the start instruction is received from the terminal 30. Then, the reception unit 15A receives an answer to the question displayed on the chat screen from the terminal 30. At this time, the change unit 15C changes one or more components corresponding to the information related to the policy among the components of the identified policy model on the basis of the answer to the question. Then, the change unit 15C generates a policy model including the diagram in which the one or more components are changed.

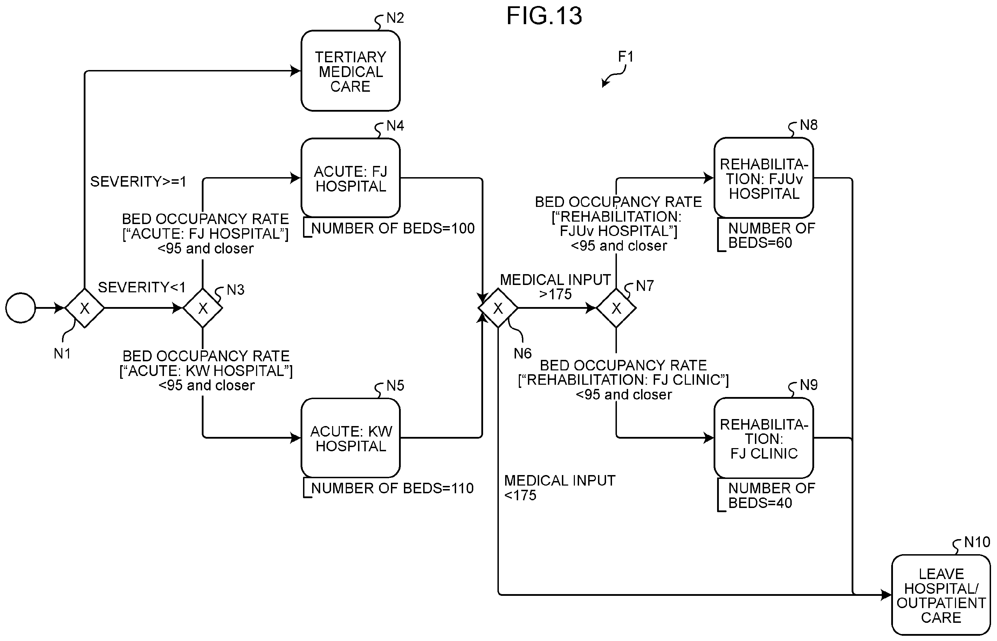

FIG. 13 is a diagram illustrating one example of a policy flow generation result. FIG. 13 illustrates a policy flow F1 generated by customizing the flow template f1 in accordance with the input results of the answers exemplified on the answer confirmation window 250 illustrated in FIG. 12.

For example, according to the answer confirmation window 250 illustrated in FIG. 12, as answers to the question template "How many acute hospitals are there?" illustrated in FIG. 4, answers on two acute hospitals, "FJ Hospital" and "KW Hospital", are acquired. In this case, the related element #1 in which association of the question template "How many acute hospitals are there?" is written is referenced, so that the component n4 and the component n5 corresponding to the service "Acute hospital" in the flow template f1 are identified as the target elements. Furthermore, the related element #1 corresponds to the modification pattern P1, so that the number of the acute hospitals arranged in parallel on the flow template f1 is customized into two.

In addition, according to the answer confirmation window 250 illustrated in FIG. 12, as answers to the question template "How many rehabilitation hospitals are there?" illustrated in FIG. 4, answers on two rehabilitation hospitals, "FJUv Hospital" and "FJ Clinic", are acquired. In this case, the related element #2 in which association of the question template "How many rehabilitation hospitals are there?" is written is referenced, so that the component n8 and the component n9 corresponding to the service "Rehabilitation hospital" in the flow template f1 are identified as the target element. Furthermore, the related element #2 corresponds to the modification pattern P1, so that the number of the rehabilitation hospitals arranged in parallel on the flow template f1 is customized into two.

Furthermore, according to the answer confirmation window 250 illustrated in FIG. 12, as answers to the question template "What is the name of the hospital?" illustrated in FIG. 4 and the question template "Which is the medical function?" illustrated in FIG. 4, the hospital name "FJ Hospital" and the medical function "Acute", the hospital name "KW Hospital" and the medical function "Acute", the hospital name "FJUv Hospital" and the medical function "Rehabilitation", and the hospital name "FJ Clinic" and the medical function "Rehabilitation" are acquired. The related element #3 and the related element #4 in which association of these question templates is written are referenced, so that the component n4 and the component n5 corresponding to the service "Acute hospital" and the component n8 and the component n9 corresponding to the service "Rehabilitation hospital" in the flow template f1 are identified as the target elements. Furthermore, the related element #3 and the related element #4 correspond to the modification pattern P1. Thus, the respective hospital names of the component n4 and the component n5 corresponding to the medical function "Acute" are changed into "FJ Hospital" and "KW Hospital", and the respective hospital names of the component n8 and the component n9 corresponding to the medical function "Rehabilitation" are changed into "FJUv Hospital" and "FJ Clinic".

Furthermore, according to the answer confirmation window 250 illustrated in FIG. 12, as answers to the question template "How many beds are there?" illustrated in FIG. 4, the number of beds in FJ Hospital, "100", the number of beds in KW Hospital, "110", the number of beds in FJUv Hospital, "60", and the number of beds in FJ Clinic, "40", are acquired. The related element #5 in which association of this question template is written is referenced, so that the meta-information on the component n4 corresponding to FJ Hospital, the meta-information on the component n5 corresponding to KW Hospital, the meta-information on the component n8 corresponding to FJUv Hospital, and the meta-information on the component n9 corresponding to FJ Clinic in the flow template f1 are identified as the target elements. Furthermore, the related element #5 corresponds to the modification pattern P2. Thus, the number of beds contained in the meta-information on the component n4 corresponding to FJ Hospital is changed into "100". Furthermore, the number of beds contained in the meta-information on the component n5 corresponding to KW Hospital is changed into "110". Furthermore, the number of beds contained in the meta-information on the component n8 corresponding to FJUv Hospital is changed into "60". Furthermore, the number of beds contained in the meta-information on the component n9 corresponding to FJ Clinic is changed into "40".

Furthermore, according to the answer confirmation window 250 illustrated in FIG. 12, as an answer to the question template "How much bed percentage can each hospital accept patients?" illustrated in FIG. 4, the bed occupancy rate "95%" is acquired. The related element #6 in which association of this question template is written is referenced, so that the parameter of the bed occupancy rate of the conditional branch component n3 and the parameter of the bed occupancy rate of the conditional branch component n7 in the flow template f1 are identified as the target elements. Furthermore, the related element #6 corresponds to the modification pattern P3. Thus, the parameter of the bed occupancy rate of the conditional branch component n3 is changed into "95%", and the parameter of the bed occupancy rate of the conditional branch component n7 is changed into "95%".

Furthermore, according to the answer confirmation window 250 illustrated in FIG. 12, as an answer to a question generated from the question template "Who are the stakeholders and what are their respective roles?" illustrated in FIG. 4, whether a tertiary medical care needs to be considered, "true", is acquired. The related element #0 in which association of this question template is written is referenced, so that the service "Tertiary medical care" component n2 and the conditional branch component n1 allocating an object to the service "Tertiary medical care" component n2 in the flow template f1 are identified as the target elements. Furthermore, the related element #0 corresponds to the modification pattern P4. Thus, the service "Tertiary medical care" component n2 and the conditional branch component n1 remain without being deleted from the flow template f1. Note that, in the example here, whether a tertiary medical care needs to be considered, "true", is acquired; however, if "false" is acquired for whether a tertiary medical care needs to be considered, the service "Tertiary medical care" component n2 and the conditional branch component n1 identified as the target elements are deleted from the flow template f1.

By executing a series of customization of the flow template f1 in this way, the policy flow F1 illustrated in FIG. 13 is generated.

Returning to description of FIG. 1, the output unit 15D executes various types of output control. Only as an example, the output unit 15D displays the policy flow generated by customizing the flow template at the change unit 15C on the client terminal 30. To this policy flow displayed on the client terminal 30, any editing can be made through a graph editing tool or the like. Note that the output destination of the policy flow is not limited to the client terminal 30. For example, the output unit 15D can also output the policy flow generated by customizing the flow template at the change unit 15C to a back-end function, a service, an application, and the like that operate on the above data infrastructure. For example, the output unit 15D displays the diagram of the policy model generated by the change unit 15C on the chat screen for the chat of which the start instruction is received from the terminal 30.

### <Processing Flow>

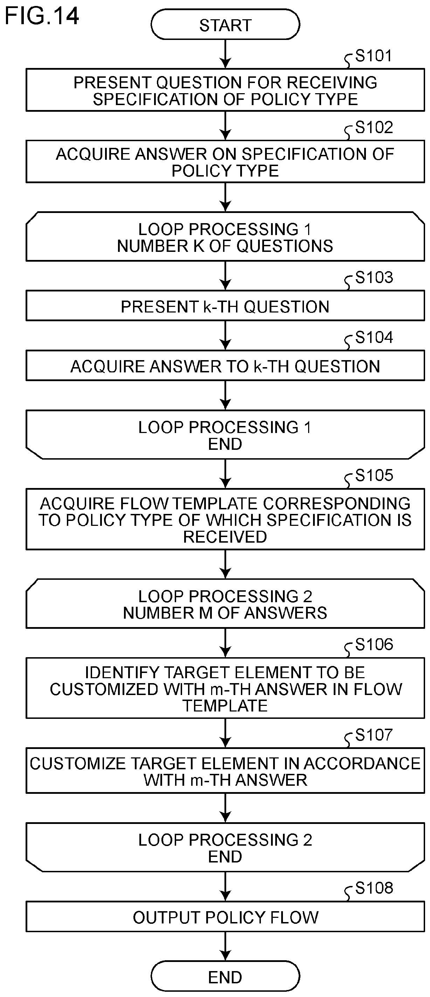

FIG. 14 is a flowchart illustrating a procedure of model output processing. Only as an example, this processing can start when the policy flow generation request is received from the client terminal 30.

As illustrated in FIG. 14, the reception unit 15A presents a question for receiving specification of a policy type to be planned, to the client terminal 30 (Step S101). Then, the reception unit 15A acquires an answer on the policy type to the question presented at Step S101 (Step S102).

Subsequently, the reception unit 15A executes loop processing 1 of repeating Step S103 and Step S104 below the number of times corresponding to the number K of questions asking about details of customization of a flow template corresponding to the policy type answered at Step S102. Note that it goes without saying that Step S103 and Step S104 can be executed in parallel for a plurality of the questions.

That is, the reception unit 15A presents a k-th question to the client terminal 30 on the basis of a question template related to the policy type answered at Step S102 in the question collection data 13A (Step S103). Then, the reception unit 15A acquires an answer to the k-th question presented at Step S103 (Step S104).

This loop processing 1 is repeated to acquire the answer to the question for each of the K questions.

Then, the identification unit 15B acquires a flow template corresponding to the policy type answered at Step S102 among the flow templates contained in the flow collection data 13B (Step S105).

Subsequently, loop processing 2 of repeating Step S106 and Step S107 below is executed the number of times corresponding to the number K of the answers acquired in the loop processing 1. Note that it goes without saying that Step S106 and Step S107 can be executed in parallel for a plurality of the answers.

That is, the identification unit 15B identifies a target element to be customized with an m-th answer in the flow template acquired at Step S105 (Step S106). Based on that, the change unit 15C customizes the target element identified at Step S106 in accordance with the modification pattern corresponding to the m-th answer (Step S107).

This loop processing 2 is repeated to execute the customization corresponding to the answer for each of the M answers to the flow template. This generates a policy flow from the flow template.

Then, the output unit 15D outputs the policy flow generated as a result of the loop processing 2 to any output destination, for example, the client terminal 30 (Step S108), and the processing ends.

### <One Aspect of Effects>

As described above, the server device 10 according to this embodiment presents a question for customizing a flow pattern in which a basic form of a policy flow is turned into a pattern and customizes part of the flow pattern on the basis of an answer to the question. Thus, a policy flow that does not lack a component indispensable for the site and meets laws, regulations, and the like can be generated. Hence, the server device 10 according to this embodiment enables generation of a policy model confirming to the site to which the policy is applied.

### <Second Embodiment>

The embodiment related to the disclosed device has been described so far; however, the present invention may be implemented in various different modes other than the above-described embodiment. Thus, another embodiment included in the present invention will be described below.

### <Chat APP>

The first embodiment above has exemplified generation of a question based on the question collection data 13A and presentation of that question to the client terminal 30; however, the question collection data 13A does not always need to be used to generate a question.

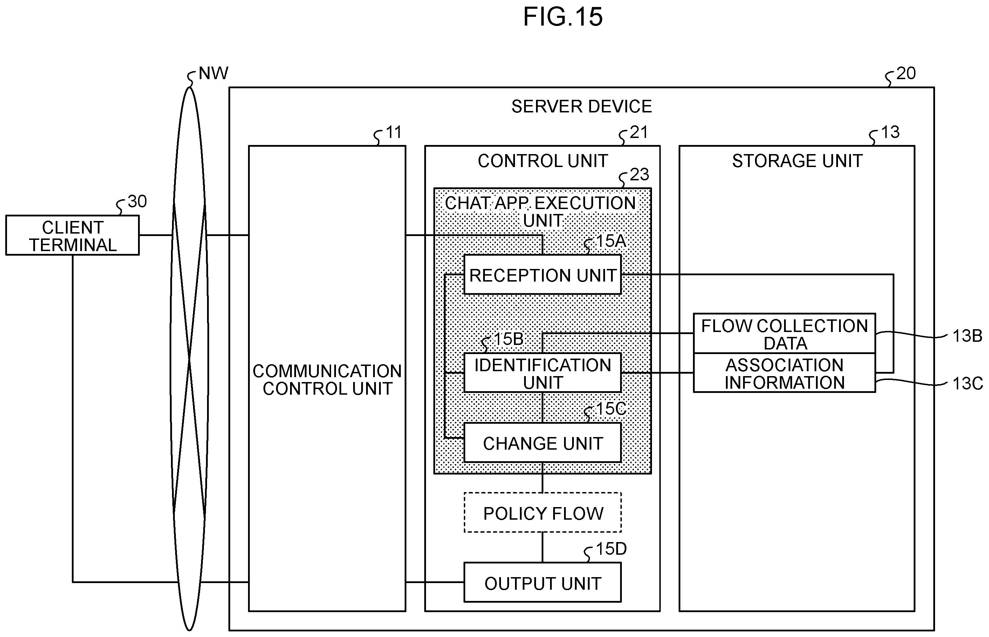

FIG. 15 is a block diagram illustrating an example functional configuration of a server device 20 according to an application example. The server device 20 illustrated in FIG. 15 differs from the server device 10 illustrated in FIG. 1 in part of the functional configuration of a control unit 21 and in that the question collection data 13A does not need to be stored in the storage unit 13. Note that, in FIG. 2, functional units that exercise the same functions as the functional units of the server device 10 are denoted by the same reference signs.

As illustrated in FIG. 15, the control unit 21 includes a chat application (APP) execution unit 23 that exercises the same functions as the reception unit 15A, the identification unit 15B, and the change unit 15C of the control unit 15 of the server device 10 illustrated in FIG. 1.

This chat APP execution unit 23 can be implemented by executing an application computer program of an AI chat implemented by a large language model, so-called LLM, such as Transformer.

In this case, by providing a prompt for the association information 13C to the chat APP, the chat APP execution unit 23 can present a question equivalent to the question presented by the reception unit 15A illustrated in FIG. 1 to the client terminal 30 even without the question collection data 13A.

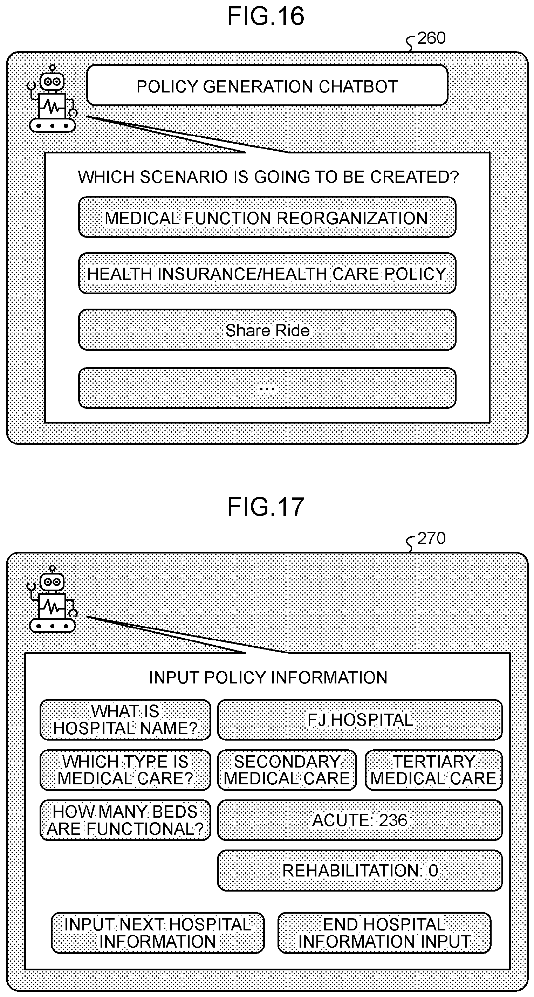

FIGS. 16 and 17 are diagrams (1) and (2) illustrating examples of a chatbot screen. FIGS. 16 and 17 illustrate chatbot screens 260 and 270 generated by the chat APP. For example, according to the chatbot screen 260 illustrated in FIG. 16, it is clear that questions equivalent to those on the question window 200 illustrated in FIG. 7 can be presented. Furthermore, according to the chatbot screen 270 illustrated in FIG. 17, it is clear that questions similar to those on the question window 210 illustrated in FIG. 8 can be presented. Note that, although the description is omitted here, chatbot screens that can present questions equivalent to those on the question windows 220 to 250 illustrated in FIGS. 9 to 11 can also be generated.

In this way, by providing a prompt for the association information 13C to the chat APP, the need for the question collection data 13A can be eliminated.

### <Dispersion and Integration>

Furthermore, each constituent of each device illustrated in the drawings does not exactly need to be physically configured as illustrated in the drawings. That is, the specific modes of dispersion/integration of the devices are not limited to those illustrated in the drawings, and all or some thereof can be configured in a functionally or physically dispersed/integrated manner in any units in accordance with various loads, usage conditions, and the like. For example, the reception unit 15A, the identification unit 15B, the change unit 15C, or the output unit 15D may be connected via a network as a device external to the server device 10 or the server device 20. Furthermore, the reception unit 15A, the identification unit 15B, the change unit 15C, and the output unit 15D may be included in the respective different devices and connected to a network for collaboration to implement the functions of the server device 10 or the server device 20.

### <Hardware Configuration>

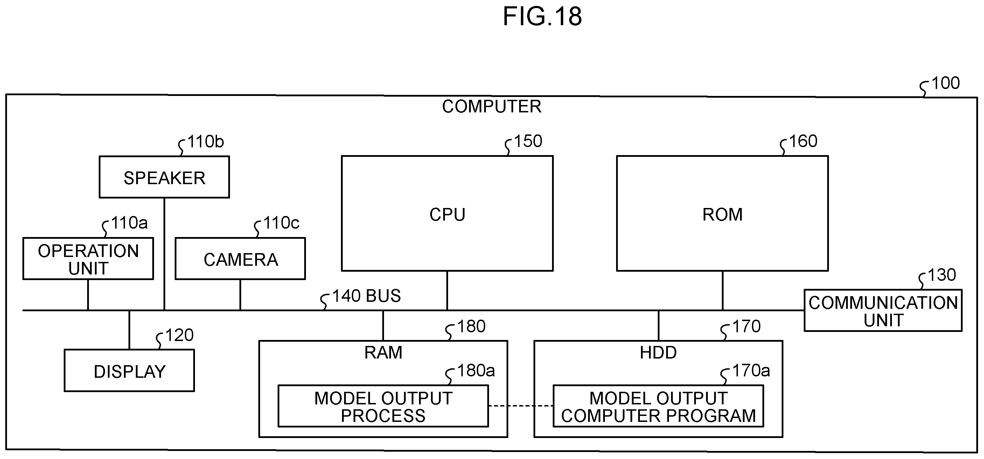

Furthermore, various types of processing described in the above embodiments can be implemented by executing a preliminarily prepared computer program on a computer, such as a personal computer and a workstation. Thus, an example computer executing a model output program having functions similar to the first embodiment and the second embodiment will be described below with reference to FIG. 18.

FIG. 18 is a diagram illustrating an example hardware configuration. As illustrated in FIG. 18, a computer 100 includes an operation unit 110a, a speaker 110b, a camera 110c, a display 120, and a communication unit 130. This computer 100 further includes a CPU 150, a ROM 160, an HDD 170, and a RAM 180. These constituents 110 to 180 are connected via a bus 140.

As illustrated in FIG. 18, the HDD 170 stores therein a model output program 170a exercising functions similar to the reception unit 15A, the identification unit 15B, the change unit 15C, and the output unit 15D described in the first embodiment above. Similar to the constituents, the reception unit 15A, the identification unit 15B, the change unit 15C, and the output unit 15D illustrated in FIG. 1, this model output program 170a may be integrated or separated. That is, the HDD 170 does not exactly need to store all pieces of the data described in the first embodiment above, and data used for the processing may be stored in the HDD 170.

Under this environment, the CPU 150 reads the model output program 170a from the HDD 170 and loads the model output program 170a in the RAM 180. As a result, the model output program 170a functions as a model output process 180a as illustrated in FIG. 18. This model output process 180a loads various pieces of data read from the HDD 170 in an area allocated to the model output process 180a in a storage area of the RAM 180 and executes various pieces of processing using the various pieces of data loaded. For example, examples of the processing executed by the model output process 180a include the processing illustrated in FIG. 14, and the like. Note that not all the processing units described in the first embodiment above need to operate on the CPU 150, and a processing unit corresponding to processing to be executed may be implemented virtually.

Note that the above model output program 170a does not always need to be stored in the HDD 170 or the ROM 160 from the beginning. For example, each computer program is stored in a "portable physical medium", such as a flexible disk, a so-called FD, a CD-ROM, a DVD disc, a magneto-optical disk, and an IC card, that is inserted into the computer 100. Then, the computer 100 may acquire each computer program from these portable physical media and execute the computer program. Alternatively, each computer program may be stored in another computer or a server device connected to the computer 100 via a public network, the Internet, a LAN, a WAN, or the like, and the computer 100 may acquire each computer program from these and execute the computer program.

### Reference Signs List

- 10: SERVER DEVICE
- 11: COMMUNICATION CONTROL UNIT
- 13: STORAGE UNIT
- 13A: QUESTION COLLECTION DATA
- 13B: FLOW COLLECTION DATA
- 13C: ASSOCIATION INFORMATION
- 15: CONTROL UNIT
- 15A: RECEPTION UNIT
- 15B: IDENTIFICATION UNIT
- 15C: CHANGE UNIT
- 15D: OUTPUT UNIT
- 30: CLIENT TERMINAL

## Claims

1. An output program causing a computer to execute processing comprising:
receiving information related to a policy;
identifying a policy model by using the received information related to the policy, the policy model being composed of a plurality of components defining a detail related to the policy in a hierarchical structure;
generating a policy model in which one or more components corresponding to the information related to the policy among the components of the identified policy model are changed; and
outputting the generated policy model.

2. The output program according to claim 1, wherein
the processing of identifying the policy model identifies a policy model including a diagram composed of a plurality of components defining a detail related to the policy in a hierarchical structure,
the generating processing identifies one or more components corresponding to the information related to the policy among the components of the identified policy model and generates a policy model including the diagram, the identified one or more components being changed based on the received information related to the policy in the policy model, and
the outputting processing displays the diagram of the generated policy model on a screen.

3. The output program according to claim 1, wherein
the identified policy model is a policy model including a diagram composed of the components,
the receiving processing includes processing of receiving a start instruction of a chat from a terminal and receiving an answer to a question from the terminal, the question asking about a detail of customization of the identified policy model and being displayed on a chat screen for the chat of which the start instruction is received from the terminal,
the generating processing includes processing of generating a policy model including the diagram, the one or more components being changed based on the answer to the question in the policy model, and
the outputting processing includes processing of displaying the diagram of the generated policy model on the chat screen for the chat of which the start instruction is received from the terminal.

4. The output program according to claim 1, wherein
the receiving processing includes processing of presenting a question based on a preliminarily arranged question template and acquiring an answer to the question,
the processing of identifying the policy model identifies a flow template corresponding to the acquired answer among preliminarily prepared flow templates and includes processing,
the generating processing includes processing of identifying one or more components corresponding to the acquired answer among a plurality of components of the identified flow template based on the identified flow template and association information in which the question template and the flow template are associated and processing of generating a policy flow graph in which the one or more components in the identified flow template are changed based on the acquired answer, and
the outputting processing includes processing of displaying the generated flow graph of a policy flow.

5. The output program according to claim 4, further causing the computer to execute processing of receiving a change in the displayed flow graph of the policy flow.

6. The output program according to claim 4, wherein the generating processing includes processing of changing a service component or a parameter of the component, the component being arranged in parallel on the flow template.

7. The output program according to claim 4, wherein the generating processing includes processing of changing meta-information on a service component included in the flow template.

8. The output program according to claim 4, wherein the generating processing includes processing of changing a parameter of a conditional branch component included in the flow template.

9. The output program according to claim 4, wherein the generating processing includes processing of deleting a conditional branch component included in the flow template, or a conditional branch component included in the flow template and a service component connected to the component.

10. An output method comprising causing a computer to execute processing including:
receiving information related to a policy;
identifying a policy model by using the received information related to the policy, the policy model being composed of a plurality of components defining a detail related to the policy in a hierarchical structure;
generating a policy model in which one or more components corresponding to the information related to the policy among the components of the identified policy model are changed; and
outputting the generated policy model.

11. An information processing device comprising a control unit configured to execute processing including:
receiving information related to a policy;
identifying a policy model by using the received information related to the policy, the policy model being composed of a plurality of components defining a detail related to the policy in a hierarchical structure;
generating a policy model in which one or more components corresponding to the information related to the policy among the components of the identified policy model are changed; and
outputting the generated policy model.
